(19) Europäisches Patentamt
European Patent Office
Office européen des brevets

(11) Numéro de publication : **0 382 617 B1**

(12) **FASCICULE DE BREVET EUROPEEN**

(45) Date de publication du fascicule du brevet : **12.08.92 Bulletin 92/33**

(51) Int. Cl.$^5$ : **C07C 321/04,** C07C 319/02

(21) Numéro de dépôt : **90400303.5**

(22) Date de dépôt : **05.02.90**

(54) **Synthèse d'alcanedithiols vicinaux.**

(30) Priorité : **08.02.89 FR 8901635**

(43) Date de publication de la demande : **16.08.90 Bulletin 90/33**

(45) Mention de la délivrance du brevet : **12.08.92 Bulletin 92/33**

(84) Etats contractants désignés : **AT BE CH DE ES FR GB GR IT LI LU NL SE**

(56) Documents cités :
**BE-A- 668 463**
**CH-A- 443 273**

(73) Titulaire : **SOCIETE NATIONALE ELF AQUITAINE (PRODUCTION)**
**Tour Elf, 2, Place de la Coupole, La Défense 6**
**F-92400 Courbevoie (FR)**

(72) Inventeur : **Vallee, Yannick**
**23, Chemin du Clos-Beaumois**
**F-14000 Caen (FR)**
Inventeur : **Labat, Yves**
**35 bis, Rue Michel Houreau**
**F-64000 Pau (FR)**

(74) Mandataire : **Leboulenger, Jean et al**
**ATOCHEM Département Propriété Industrielle**
**F-92091 Paris la Défense 10 Cédex 42 (FR)**

EP 0 382 617 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la fabrication d'alcanedithiols vicinaux et, plus particulièrement, celle de l'éthanedithiol à partir du trithiocarbonate d'éthylène.

L'éthanedithiol $HS-CH_2CH_2-SH$ est un important mercaptan qui a trouvé diverses applications industrielles, notamment comme intermédiaire de synthèse et comme agent de vulcanisation. Il peut être préparé en traitant l'acétate de mercaptoéthyle par le sulfhydrate d'ammonium à chaud et sous pression d'hydrogène sulfuré (brevet FR 2 102 936). On peut également l'obtenir à partir du dibromoéthane par action de la thiourée puis de la potasse (Organic Synthesis, Coll. Vol. IV, 1963, p. 401). Par ailleurs, le brevet belge 668 463 décrit sa synthèse par action du trithiocarbonate de sodium sur le dichloroéthane ; toutefois, dans les conditions décrites, on obtient un mélange dont l'éthanedithiol n'est isolé qu'avec un rendement de 38 %.

D'anciennes méthodes permettant d'obtenir l'éthanedithiol à partir du trithiocarbonate d'éthylène :

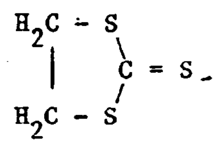

sont décrites dans la littérature (cf. E.E. Reid, Organic Chemistry of Bivalent Sulfur, Vol. I, Chemical Publishing, New-York, 1958, pp. 41-42). Elles consistent à traiter le trithiocarbonate d'éthylène par une base, à chaud, et souvent sous pression. Les rendements de ces réactions sont généralement très faibles et peu reproductibles ; il se forme des quantités importantes, parfois même majoritaires, de sous-produits :

– sulfure de dimercaptoéthyle : $HS-CH_2CH_2-S-CH_2CH_2-SH$

– oligomères et polymères lourds de l'éthanedithiol.

T.Taguchi et al. (Tetrahedron Letters 41, 1969, pp. 3631-4) ont obtenu l'éthanedithiol en traitant le trithiocarbonate d'éthylène par l'éthanolamine à 80-120°C. Malgré sa commodité, cette méthode présente l'inconvénient de produire une masse importante de sous-produits organiques difficilement récupérables. Quant aux méthodes de réduction par $AlLiH_4$ (S.M. Iqbal et al, J. Chem. Soc., 1960, p. 1030), leur coût est industriellement prohibitif.

Le trithiocarbonate d'éthylène est un produit d'accès facile ; il peut, par exemple, être obtenu quantitativement par action d'un tri-thiocarbonate alcalin sur le dichloroéthane. Il a maintenant été trouvé que le trithiocarbonate d'éthylène peut être avantageusement converti en éthanedithiol par action d'un sulfure alcalin, puis d'un acide conformément au schéma suivant :

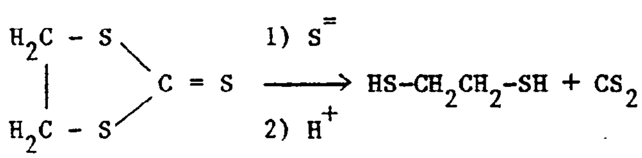


Cette méthode de préparation de l'éthanedithiol peut s'appliquer généralement à la synthèse d'alcanedithiols vicinaux à partir des trithiocarbonates d'alkylène correspondants tels que, par exemple, le trithiocarbonate de propylène conduisant au propanedithiol-1,2.

Le procédé selon l'invention est donc caractérisé en ce qu'il consiste à faire réagir un trithiocarbonate d'alkylène avec un sulfure alcalin dans un solvant, puis à acidifier le milieu réactionnel et à séparer l'alcanedithiol formé.

Le solvant utilisé pour la réaction du trithiocarbonate d'alkylène avec le sulfure alcalin peut être de l'eau. Toutefois, on obtient de meilleurs résultats, notamment une bien moindre teneur en sulfure de dimercaptoalkyle dans le produit brut final, en opérant dans un mélange d'eau et d'un solvant organique soluble dans l'eau tel que, par exemple, un alcool inférieur (1 à 4 atomes de carbone), en particulier le méthanol. On peut éventuellement opérer en l'absence d'eau, par exemple dans du méthanol pur ; les résultats ne sont toutefois pas meilleurs. De préférence, on choisira un rapport des volumes d'eau et d'alcool compris entre 90/10 et 10/90, et plus particulièrement entre 50/50 et 25/75.

La solution de sulfure alcalin (par exemple $Na_2S$ ou $K_2S$) peut être obtenue soit par absorption d'hydrogène sulfuré dans une solution de l'hydroxyde alcalin correspondant (NaOH ou KOH), soit par dissolution d'un sulfure solide commercial (en particulier $Na_2S$, $3H_2O$ ou $Na_2S$, $9H_2O$) dans le solvant. Bien qu'on puisse utiliser des solutions très diluées, il est économiquement plus avantageux d'opérer avec une solution de sulfure alcalin

aussi concentrée que possible.

La température à laquelle est effectuée la réaction du trithiocarbonate d'alkylène avec le sulfure alcalin peut varier dans de larges limites et peut aller notamment de la température ambiante jusqu'à celle de reflux du solvant (64°C dans le cas du méthanol). Il est cependant préférable d'opérer à la température de reflux ; on peut alors utiliser des solutions plus concentrées de sulfure alcalin (environ 3,5 molaire contre environ 2 molaire à la température ambiante) et minimiser ainsi la quantité de solvant à employer. On ne sortirait pas toutefois du cadre de la présente invention en opérant en-dessous de la température ambiante, par exemple avec un mélange non-homogène de solvant et de sulfure alcalin, ou sous pression à une température supérieure à celle du reflux du solvant à la pression atmosphérique.

La quantité de sulfure alcalin à employer peut être comprise entre environ un équivalent molaire par rapport au trithiocarbonate d'alkylène et un large excès, par exemple trois équivalents molaires. Il est toutefois préférable d'opérer avec un léger excès de sulfure alcalin par rapport au trithiocarbonate d'alkylène, par exemple de 1,05 à 1,6 équivalents ; on assure ainsi une conversion quasi-totale.

La réaction peut être conduite en mélangeant dès le départ tout le trithiocarbonate d'alkylène au sulfure alcalin. Toutefois, on obtient de meilleures résultats en ajoutant progressivement le trithiocarbonate d'alkylène dans la solution de sulfure alcalin portée au reflux. Dans le cas du trithiocarbonate d'éthylène (F = 35°C), il est plus commode de l'utiliser à l'état fondu, mais on ne sortirait pas du cadre de la présente invention en utilisant du trithiocarbonate d'éthylène solide. La durée d'addition peut varier dans de larges limites en fonction des quantités mises en oeuvre ; elle est le plus souvent comprise entre 5 et 120 minutes.

Lorsque la réaction est terminée, ce qui se traduit par l'homogénéisation du milieu réactionnel, on procède alors à son acidification, de préférence au moyen d'un acide minéral fort tel que, par exemple, l'acide chlorhydrique, ou l'acide sulfurique. La quantité d'acide à employer est au moins molairement équivalente à la quantité de cations alcalins ($Na^+$, $K^+$) utilisée. Afin d'obtenir un pH bien acide, il est avantageux d'utiliser un léger excès d'acide, par exemple 1,1 équivalent $H^+$ par cation $Na^+$ ou $K^+$. Il a en outre été constaté qu'on obtient de meilleurs rendements lorsque le mélange réactionnel passe rapidement d'un pH basique (pH > 10) à un pH très acide (pH = 1-2). Cette variation brutale de pH peut être obtenue en mélangeant rapidement l'acide et le milieu réactionnel ou mieux en versant le milieu réactionnel dans l'acide.

Après l'acidification, il décante une phase organique qui contient l'alcanedithiol et du disulfure de carbone. L'alcanedithiol peut être aisément séparé de cette phase, par exemple par évaporation du $CS_2$, puis distillation.

Le procédé selon l'invention permet de recycler une partie des produits utilisés. L'acidification de la solution réactionnelle transforme le léger excès de sulfure alcalin utilisé en hydrogène sulfuré ; celui-ci peut être récupéré, par exemple par barbotage dans un piège contenant un hydroxyde alcalin, pour reformer ainsi du sulfure alcalin de départ. Le disulfure de carbone, produit par l'acidification et séparé du dithiol, peut être réutilisé pour la synthèse du trithiocarbonate d'alkylène de départ. Enfin, le solvant éventuellement utilisé peut être séparé de l'eau par distillation, effectuée de préférence après l'acidification ; cette distillation peut éventuellement être faite avant l'acidification, mais on opèrera alors de préférence sous un léger vide pour ne pas chauffer inutilement les produits de la réaction.

Les exemples suivants illustrent l'invention, sans la limiter. Les pourcentages indiqués à propos des analyses CPV ne prennent en compte que l'éthanedithiol, le sulfure de dimercaptoéthyle et le trithiocarbonate d'éthylène, et sont exprimés par rapport au poids total de ces trois composés.

## EXEMPLE 1

Dans 60 ml d'eau portée au reflux, et sous agitation, on dissout 14 g (0,1056 mole) de $Na_2S, 3H_2O$, puis on ajoute en 30 minutes 9 g (0,066 mole) de trithiocarbonate d'éthylène. Cette addition terminée, on maintient sous agitation à reflux pendant encore 45 minutes.

Le mélange réactionnel est alors rapidement acidifié avec 28 ml d'acide chlorhydrique à 32 %. Il décante une phase organique dont l'analyse CPV montre qu'elle contient, hormis le disulfure de carbone, 59 % d'éthanedithiol, 36 % de sulfure de dimercaptoéthyle et 5 % de trithiocarbonate d'éthylène.

Par distillation, on récupère l'éthanedithiol avec un rendement de 48 %.

## EXEMPLE 2 (comparatif)

On dissout 2,8 g (0,0693 mole) d'hydroxyde de sodium dans un mélange de 40 ml d'eau et 80 ml de méthanol. Le tout est porté à reflux sous agitation, puis on ajoute en 30 minutes 9 g (0,066 mole) de trithiocarbonate d'éthylène. L'addition terminée, on poursuit l'agitation à reflux pendant 10 minutes, puis on acidifie rapidement le mélange réactionnel avec 10 ml d'acide chlorhydrique à 32 %.

L'analyse CPV de la phase organique décantée montre qu'elle contient, hormis le disulfure de carbone,

30 % d'éthanedithiol, 20 % de sulfure de dimercaptoéthyle et 50 % de trithiocarbonate d'éthylène.

Par distillation, on récupère l'éthanedithiol avec un rendement de 23 %.

EXEMPLE 3

Dans 20 ml d'eau et 40 ml de méthanol au reflux et sous agitation, on dissout 31,8 g (0,24 mole) de $Na_2S$, $3H_2O$, puis on ajoute en 40 minutes 27 g (0,198 mole) de trithiocarbonate d'éthylène et poursuit l'agitation pendant encore 15 minutes après la fin de l'addition.

Le mélange réactionnel est ensuite acidifié en le versant dans 60 ml d'acide chlorhydrique à 32 %. Il décante une phase organique dont l'analyse CPV montre qu'hormis le $CS_2$, elle contient 96 % d'éthanedithiol, 2 % de sulfure de dimercaptoéthyle et 2 % de trithiocarbonate d'éthylène.

Par distillation, on récupère 14 g d'éthanedithiol, soit un rendement de 75 %.

EXEMPLE 4

a) Dans un réacteur agité thermostaté de 50 litres, on charge 30 kg d'une solution aqueuse contenant 70 moles de $Na_2S$, puis 6080 g (80 moles) de disulfure de carbone. Après avoir porté le mélange à la température d'ébullition du $CS_2$, on l'y maintient pendant 3 heures, puis on élimine l'excès de $CS_2$ par évaporation.

On introduit alors dans le milieu réactionnel 6039 g (61 moles) de dichloroéthane de telle façon que l'exothermicité de la réaction puisse être contrôlée et que la température du milieu ne dépasse pas 80°C. Cette opération dure environ 3 heures. On décante ensuite la phase organique vers 40°C. Elle est essentiellement constituée de trithiocarbonate d'éthylène (8296 g).

b) Dans le même réacteur, on introduit une solution de 70 moles de $Na_2S$, $3H_2O$ dans un mélange de 9760 g de méthanol et 5490 g d'eau, puis on porte à l'ébullition du méthanol et introduit alors régulièrement en une heure les 8296 g de trithiocarbonate d'éthylène brut précédemment obtenus. On poursuit la réaction pendant 2 heures à reflux, puis on enlève du réacteur la solution homogène ainsi obtenue (solution 1).

c) Dans le même réacteur, on charge 30 kg d'une solution aqueuse contenant 144 moles de HCl, puis on y introduit régulièrement la solution 1 de façon à contrôler l'exothermicité. L'éthanedithiol et le $CS_2$ formés sont ensuite séparés de la phase aqueuse par décantation, puis le $CS_2$ est aisément séparé de l'éthanedithiol brut par évaporation sous vide.

On obtient ainsi un produit dont la composition pondérale, déterminée par analyse CPV, est la suivante :

93 % d'éthanedithiol,
5 % de disulfure de dimercaptoéthyle,
2 % de trithiocarbonate d'éthylène.

Par distillation de ce produit, on obtient 4020 g d'éthanedithiol d'une pureté supérieure à 99 %, ce qui correspond à un rendement de 70 % par rapport au dichloroéthane.

## Revendications

1. Procédé de préparation d'alcanedithiois vicinaux, caractérisé en ce qu'il consiste à faire réagir un trithiocarbonate d'alkylène avec un sulfure alcalin dans un solvant, puis à acidifier le milieu réactionnel et à séparer l'alcanedithiol formé.

2. Procédé selon la revendication 1, dans lequel le solvant est de l'eau ou un solvant organique soluble dans l'eau, de préférence un alcool inférieur.

3. Procédé selon la revendication 1, dans lequel le solvant est un mélange d'eau et d'alcool, de préférence le méthanol, le rapport des volumes d'eau et d'alcool étant compris entre 90/10 et 10/90 et, de préférence, entre 50/50 et 25/75.

4. Procédé selon l'une des revendications 1 à 3, dans lequel la réaction est effectuée à une température allant de la température ambiante à celle de reflux du solvant, et de préférence à cette dernière.

5. Procédé selon l'une des revendications 1 à 4, dans lequel on utilise de 1 à 3 moles de sulfure alcalin par mole de trithiocarbonate d'alkylène, et de préférence 1,05 à 1,6.

6. Procédé selon l'une des revendications 1 à 5, dans lequel on utilise du sulfure de sodium.

7. Procédé selon l'une des revendications 1 à 6, dans lequel le trithiocarbonate d'alkylène est ajouté progressivement à la solution de sulfure alcalin.

8. Procédé selon l'une des revendications 1 à 7, dans lequel l'acidification est réalisée avec un acide minéral, de préférence l'acide chlorhydrique.

9. Procédé selon l'une des revendications 1 à 8, dans lequel l'acidification est réalisée en versant le

mélange réactionnel basique dans l'acide.

10. Procédé selon l'une des revendications 1 à 9, dans lequel on prépare l'éthanedithiol à partir du trithiocarbonate d'éthylène.

11. Procédé selon l'une des revendications 1 à 9, dans lequel on prépare le propanedithiol-1,2 à partir du trithiocarbonate de propylène.

## Patentansprüche

1. Verfahren zur Herstellung von vicinalen Alkandithiolen, dadurch gekennzeichnet, daß man ein Alkylentrithiocarbonat mit einem Alkalisulfid in einem Lösungsmittel reagieren lässt, anschließend das Reaktionsmedium ansäuert und das gebildete Alkandithiol abtrennt.

2. Verfahren nach Anspruch 1, in dem das Lösungsmittel Wasser oder ein wasserlösliches organisches Lösungsmittel, vorzugsweise ein niederer Alkohol, ist.

3. Verfahren nach Anspruch 1, in dem das Lösungsmittel ein Gemisch aus Wasser und Alkohol, vorzugsweise Methanol, ist, wobei das Volumenverhältnis von Wasser zu Alkohol zwischen 90/10 und 10/90, und vorzugsweise zwischen 50/50 und 25/75 liegt.

4. Verfahren nach einem der Ansprüche 1 bis 3, in dem die Reaktion bei einer Temperatur durchgeführt wird, die von Raumtemperatur bis zur Rückflußtemperatur des Lösungsmittels geht, und vorzugsweise bei letzterer liegt.

5. Verfahren nach einem der Ansprüche 1 bis 4, in dem man 1 bis 3, vorzugsweise 1,05 bis 1,6 Mol Alkalisulfid pro Mol Alkylentrithiocarbonat verwendet.

6. Verfahren nach einem der Ansprüche 1 bis 5, in dem man Natriumsulfid verwendet.

7. Verfahren nach einem der Ansprüche 1 bis 6, in dem das Alkylentrithiocarbonat der Alkalisulfidlösung nach und nach zugefügt wird.

8. Verfahren nach einem der Ansprüche 1 bis 7, in dem die Ansäuerung mit einer Mineralsäure, vorzugsweise Salzsäure, durchgeführt wird.

9. Verfahren nach einem der Ansprüche 1 bis 8, in dem die Ansäuerung durch Eingießen des basischen Reaktionsgemisches in die Säure durchgeführt wird.

10. Verfahren nach einem der Ansprüche 1 bis 9, in dem man Ethandithiol aus Ethylentrithiocarbonat herstellt.

11. Verfahren nach einem der Ansprüche 1 bis 9, in dem man 1,2-Propandithiol aus Propylentrithiocarbonat herstellt.

## Claims

1. Process for the preparation of vicinal alkanedithiols, characterised in that it consists in reacting an alkylene trithiocarbonate with an alkali metal sulphide in a solvent and then acidifying the reaction mixture and separating off the alkanedithiol formed.

2. Process according to Claim 1, in which the solvent is water or a water-soluble organic solvent, preferably a lower alcohol.

3. Process according to Claim 1, in which the solvent is a mixture of water and alcohol, preferably methanol, the ratio of the volumes of water and alcohol being between 90/10 and 10/90 and preferably between 50/50 and 25/75.

4. Process according to one of Claims 1 to 3, in which the reaction is carried out at a temperature ranging from room temperature to that of the solvent reflux, and preferably at the latter.

5. Process according to one of Claims 1 to 4, in which from 1 to 3 moles of alkali metal sulphide are employed per mole of alkylene trithiocarbonate, and preferably 1.05 to 1.6.

6. Process according to one of Claims 1 to 5, in which sodium sulphide is employed.

7. Process according to one of Claims 1 to 6, in which the alkylene trithiocarbonate is added gradually to the alkali metal sulphide solution.

8. Process according to one of Claims 1 to 7, in which the acidification is carried out with an inorganic acid, preferably hydrochloric acid.

9. Process according to one of Claims 1 to 8, in which the acidification is carried out by pouring the basic reaction mixture into the acid.

10. Process according to one of Claims 1 to 9, in which ethanedithiol is prepared from ethylene trithiocarbonate.

11. Process according to one of Claims 1 to 9, in which 1,2-propanedithiol is prepared from propylene trithiocarbonate.